# NEW EUROPEAN PATENT SPECIFICATION

(11) **EP 1 842 564 B2**
(45) Date of publication and mention of the opposition decision: **18.11.2026**
(45) Mention of the grant of the patent: 19.02.2014
(21) Application number: 06016651.9
(22) Date of filing: 09.08.2006
(51) Int. Cl.: A61L 15/46

(54) **Absorbent articles including odour control system**
Absorbierender Artikel mit einem Geruchskontrollsystem
Articles absorbant avec un système de contrôle des odeurs

(30) Priority: 05.04.2006 EP 06112262
(43) Date of publication of application: 10.10.2007
(60) Divisional application: 10178628.3 / 2 258 408
(73) Proprietor: The Procter & Gamble Company, Cincinnati, OH 45202 (US)
(72) Inventor: Carlucci, Giovanni, 66100 Chieti (IT); Pesce, Antonella, 00100 Roma (IT); Caputi, Mariangela, 70056 Molfetta (Ban) (IT); Sierri, Giancarlo, 64030 Montefino (Teramo) (IT); Gagliardini, Alessandro, 64100 Villa Vomana (Teramo) (IT)
(74) Representative: P&G Patent Germany

(56) References cited:
- EP-A1- 1 275 404
- WO-A1-01/79409
- WO-A1-97/12575
- US-A- 5 807 587
- US-A1- 2001 028 126
- US-A1- 2003 200 936
- US-A1- 2004 037 792
- US-A1- 2004 037 792
- US-A1- 2005 203 473
- DATABASE WPI Section Ch Week 199630, Derwent World Patents Index; Class D22, AN 1996-295491, XP002399310
- Caren D. Frizzo et al., "Essential Oils of Camphor Tree (Cinnamomumcamphora Nees & Eberm) Cultivated in Southern Brazil", Brazilian Archivesof Biology and Technology, ISSN 1516-8913, Braz. arch. biol. technol. vol.43no. 3 Curitiba 2000, published in 2000
- Indu Sasidharan and A. Nirmala Menon, "COMPARATIVE CHEMICALCOMPOSITION AND ANTIMICROBIAL ACTIVITY FRESH & DRY GINGEROILS (ZINGIBER OFFICINALE ROSCOE)", International Journal ofCURRENT PHARMACEUTICAL RESEARCH, Vol 2, Issue 4, 2010, pp.40-43
- Paul Chidozie Onyenekwe and Seiji Hashimoto, "The composition of the essential oil of dried Nigerian ginger (Zingiber officinale Roscoe)”, Eur FoodRes Technol (1999) 209, pp. 407-410;
- Zhu Liangfeng et al., "Aromatic Plants and Essential Constituents Supplement 1" (p. 61), South China Institute of Botany, Chinese Academy of Sciences, Hai Feng Publishing Co., Chinese National Node for APINMAP, distributed by Peace Book Co. Ltd., Hong Kong (1995

## Description

### FIELD OF THE INVENTION

The present invention relates to absorbent articles which are provided with an odour control system. The odour control system comprises classes of odour control materials, wherein one class reduces malodours by acting on the malodours or malodorous substance and a second class acts on nose receptors. The classes of odour control materials may be selected to provide a synergistic effect in terms of malodour reduction.

### BACKGROUND OF THE INVENTION

Absorbent articles of personal hygiene are known in the art. Typical examples include sanitary napkins, panty liners, adult incontinence articles, infant diapers, paper towels, bath tissue and facial tissue. Such articles are often used to absorb and retain bodily fluids and other exudates excreted by the human body. Typically, such exudates are perceived as malodorous and offensive. Therefore, methods and materials for controlling and reducing malodours in absorbent articles have been developed. Some examples are discussed hereinafter.

An early, basic reference in this respect is EP 510619. This document discloses a wide variety of materials, which have proven to be effective in certain circumstances in reducing malodours in absorbent articles of personal hygiene. EP 959846 discloses such materials comprising polyacrylate superabsorbers and silica. EP 811387 discloses absorbent articles being provided with a zeolite and silica odour control system. EP 963186 discloses an odour control systems comprising zeolites, silica and polyacrylic superabsorbers. EP 912149 discloses chelating agents for use in odour control in absorbent articles, particularly polyfunctionally substituted aromatic chelants.

US2001/028126 discloses a method for making an odor absorbent catamenial/tampon product. The odor adsorbent materials that can be used in the process include aldehydes, sensory receptor alterants, masking agents, etc. or any mixtures thereof. The aldehydes enter into chemical reaction with malodorant molecules. Examples for suitable aldehydes are e.g. decanal, benzaldehyde, vanillin. Further compounds can be used, e.g. natural oils to impart a pleasant odor which overpowers the malodor; masking agents to mask or hide the malodor; compounds that are capable of altering the body's sensory receptors.

All of the above solutions can provide a consumer-noticeable degree of malodour reduction in absorbent articles. However, due to the nature of action and the materials chosen only a limited variety of malodorous compounds can be counteracted.

Therefore it is desirable to provide absorbent articles having an odour control system, which acts against a wide variety of malodours in a holistic manner. It is also desirable to provide an absorbent article having an odour control system including at least one class of materials that reduces malodours by acting on malodours or a malodourous substance in the article and/or at least one class that acts on certain nose receptors. It would also be desirable to provide an absorbent article including an odour control system having at least one material that acts on the malodours and/or malodorous substance to reduce the odour and the same or another material that acts on nose receptors to help reduce the perception of malodour.

### SUMMARY OF THE INVENTION

The present invention addresses the above need by providing an odour control system, which provides an absorbent article comprising an odour control system, wherein the odour control composition comprises at least two classes of odour control materials. The first class of odour control material reduces odour by acting on malodours or a malodorous substance in the absorbent article and a second class of odour control material reduces odour by acting on the user's nose receptors. The absorbent article is characterized in that the first class of odour control material is selected from aldehydes selected from the group consisting of alpha-amylcinnamic aldehyde in an amount of from 0.05 g/m² to 20 g/m², and the second class of odour control material is selected from the group consisting of menthyl acetate in an amount of from 0.05 g/m² to 20 g/m².

### DET AILED DESCRIPTION OF THE INVENTION

An "absorbent article" is able to receive and/or absorb and/or contain and/or retain fluids and/or exudates, especially bodily fluids/bodily exudates. Absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner) Disposable absorbent articles according to the present invention are diapers, surgical and wound dressings and perspiration pads, incontinence pads, as well as sanitary napkins, panty liners, tampons for feminine hygiene and interlabial devices. Certain absorbent articles include a fluid pervious topsheet, a fluid impervious backsheet that may be water vapour and/or gas pervious and an absorbent core comprised there between.

The term "use", as used herein, refers to the period of time that starts when the absorbent article is actually put in contact with the anatomy of a wearer. It is further underlined that the odour neutralization benefits provided by the absorbent articles of the present invention are already provided as soon as the absorbent articles is released from its package. This is due to the volatility of the odour neutralization materials acting on nose receptors (sometimes referred to herein as acting "externally"), which are further specified below.

By "body fluid" it is meant herein any fluid produced by human body including, but not limited to, perspiration, urine, menstrual fluids, vaginal secretions and the like.

The present invention relates to an odour control system, which reduces the malodour generated by a broad range of odorous materials typically occurring in or resulting from the degradation of body fluids and/or materials making up the absorbent article. The odour control system herein includes at least two classes of odour control materials; one class of odour control materials counteracts the malodours of the exudates or other malodorous material (also referred to herein as acting "internally"), whereas a second class counteracts such malodours by affecting the receptors in the nose.

### Odour control materials acting on the malodours or malodorous substance

There are many materials known in the art for counteracting malodours in absorbent articles. Examples can be found in the references cited herein before. Typical substances are zeolites, starch, activated carbon, cyclodextrine, chitin or chitosan and esters.

These actives can reduce the malodour unpleasantness according to different mechanisms, e.g. they can reduce the amount of malodorous molecules through absorption/adsorption mechanisms and/or can react with the malodorous molecules transforming them into low volatile/non-odorous ones and/or can suppress malodorous molecules volatility and/or can prevent the malodour generation by inhibiting degradative processes caused by micro-organisms metabolic activity.

For the odour control system of the present invention a specific selection of such materials is desired. It has been found that aldehydes are particularly useful.

It has further been found by the present inventors that narrow silica gel or zeolites, especially mesoporous zeolites, can be used to stabilize the volatile odour control materials acting externally in the absorbent article. Mesoporous zeolites are those zeolites with pore size from 20 to 500 Å. As indicated above, silica gel can absorb volatile substances and thus reduces its migration out of the absorbent article. Advantageously the shelf life of the absorbent articles is significantly prolonged thereby.

Without being bound by theory it is assumed that narrow silica gel can adsorb the most volatile and active compounds due to is porosity, particularly by creation of hydrogen-bonding, and easily release active compounds during usage of product, i.e. due to presence of water that competes with the absorbed molecules in the formation of hydrogen bonds with the silica gel surface.

The stabilization effect has been proven by running a Thermogravimetric analysis (TGA) on samples of narrow silica gel treated with 10 weight-% of menthyl acetate. Specifically, the method is based on evaluation of weight loss over time at specific temperature (40°C). The samples were kept at 40°C for 180 minutes. In order to have a basis for comparison the TGA analysis was performed on a sample of pure menthyl acetate and a sample of pure silica gel as references together with a sample of menthyl acetate (10%) + silica gel (ex Grace, coded 123). The results are listed in table 1 and illustrate that the sample with silica gel and MA has a significant lower weight loss than the samples of alone.

**Table 1**

| Data point | Sample composition | Weight Loss (%) |
|---|---|---|
| 1 | Menthyl acetate | ~100% |
| 2 | Silica gel + Menthyl acetate | 3 |
| 3 | Silica gel | 3 |

One component of body fluid malodour is ammonia. For example ammonia is present in high amounts in products used for urine absorption due to degradation of urea. Ammonia and its derivatives can react with aldehyde to form imines (according to the so-called Schiff base reaction).

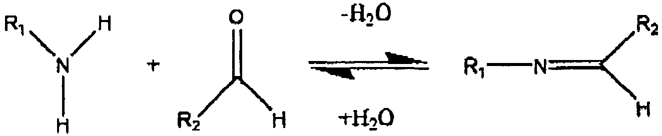

This reaction is catalyzed by enzymes and/or by a slightly acidic pH 4 to 5. The moderate acid requirement is necessary to allow protonation of the hydroxyl intermediate to allow water to leave.

Unfortunately, most aldehydes capable of imine reaction have an unpleasant and/or too intense odour that can be disturbing to human nose and/or they are very volatile and so not stable on the product.

Therefore, it is desirable to select suitable materials for controlling malodour. Examples of suitable aldehydes for controlling malodour are those aldehydes that are able to react with aminic compounds according to Schiff base reaction and have not unpleasant odour. Suitable aldehydes include alpha-amylcinnamic aldehyde, p-anisaldehyde, benzaldehyde, cinnamic aldehyde, cuminic aldehyde, decanal, p-t-butyl-alpha-methyldihydrocinnamaldehyde, 4-hydroxy-3-rnethoxycinnatnaldehyde, 2-phenyl-3-(2-furyl)prop-2-enal, vanillin isobutyrate, ethyl vanillin acetate, vanillin acetate, cyclamen aldehyde, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)-2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial and mixtures thereof.

Particularly suitable aldehydes are alpha-amylcinnamic aldehyde, decanal, 4-hydroxy-3-methoxycinnamaldehyde, 3,5-dimethoxy-4-hydroxycinnamaldehyde, 2-phenyl-3-(2-furyl)prop-2-enal, ethyl -vanillin- acetate, vanillin isobutyrate, vanillin acetate, asaronaldehyde.

The aldehydes for application herein are selected from the group consisting of alpha-amylcinnamaldehyde.

### Odour control materials acting on nose receptors

The second class of odour control materials in the odour control systems counteracts odours externally, outside the absorbent articles. Specifically, the materials listed hereinafter inhibit the receptors of the nose, hereinafter called "nose blocking". When used, these materials may significantly reduce the capability for the nose to detect the malodours.

The nose blocking is possible due to the volatile nature of the materials selected, which are evaporating out of the absorbent article and are then inhaled into the nose of an individual generally within somewhat close range of the article, e.g. within about 0 to 10 meters of the article (although this should in no way be intended to limit the scope of the invention) by normal breathing. The blocking of the nose receptors is of course only temporary.

Suitable nose blocking materials include menthol, menthyl acetate, 3-buten-2-one,3-methyl-4-(2,6,6-trimethyl-2-cyclohexen-1-yl)-, 4-(2,6,6-trimethylcyclohen-1-en-1-yl)but-3-en-2-one, 3-buten-2-one,4-(2,6,6-trimethyl-2-cyelohexen-1-yl)-,(E)-, menthyl lactate, isomenthyl acetate, isomenthyl propionate, isomenthyl isobutyrate, isomenthyl butyrate, camphor and p-menthane. The materials also include their isomeric forms, diastereomers and enantiomers. Advantageously, in generally, the above materials have only a very slight inherent odour but show a high degree of nose receptor blocking. The second class of odour control material for application herein is selected from the group consisting of methyl acetate.

### Optional further components

### a) Hydrogel forming absorbent polymers

The absorbent article of the present invention can also comprise, in addition to the odour control system, a hydrogel forming absorbent polymer. Hydrogel forming absorbent polymers useful in the present invention include a variety of water-insoluble, but water-swellable polymers capable of absorbing large quantities of fluids, and are also commonly referred to in the art as absorbent gelling materials, or superabsorbent materials.

Absorbent gelling materials, as it is known in the art, are widely used to provide absorbent articles with improved absorbent capacity, being typically comprised in absorbent structures, alone or in combination with fibrous absorbent material such as for example pulp, airfelt, tissue or nonwoven layers. Absorbent gelling materials are also used in combination with known odour control materials, as they can provide additional benefit in odour control capability together with their liquid absorption capacity.

Absorbent gelling materials form hydrogels on contact with water (e.g., with urine, blood, and the like). One suitable type of hydrogel-forming, absorbent gelling material is based on polyacids, especially polyacrylic acid. Hydrogel-forming polymeric materials of this type are those which, upon contact with fluids (i.e., liquids) such as water or body fluids, imbibe such fluids and thereby form hydrogels. These absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, suitable absorbent gelling materials include hydrolyzed acrylonitrile grafted starch, acrylic acid grafted starch, polyacrylates, maleic anhydride-based copolymers and combinations thereof. Particularly suitable absorbent gelling materials are the polyacrylates and acrylic acid grafted starch.

Whatever the nature of the polymer components of the absorbent gelling materials, such materials will in general be slightly cross-linked. Crosslinking serves to render these hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Suitable cross-linking agents are the di- or polyesters of unsaturated mono- or polycarboxylic acids with polyols, the bisacrylamides and the di-or triallyl amines. Particularly suitable cross-linking agents are N, N'-methylenebisacrylamide, trimethylol propane triacrylate and triallyl amine.

The slightly cross-linked, hydrogel-forming absorbent gelling materials can generally be employed in their partially neutralized form. For purposes described herein, such materials are considered partially neutralized when at least 25 mole percent, and typically at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The absorbent gelling materials herein before described can be typically used in the form of discrete particles Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For example, particle sizes-comprised between 150 µ and 800 µ are commonly used in the art, although smaller or greater particle sizes can be also used. "Particle Size" as used herein means the weighted average of the smallest dimension of the individual particles.

According to an embodiment of the present invention, the absorbent gelling material which may be comprised in the absorbent article is selected such as to have a particularly high permeability or flow conductivity when swollen with body fluids forming a hydrogel zone or layer. This is particularly advantageous when the absorbent gelling material is provided in relatively high concentration in order to provide a high absorption capacity in absorbent articles, for example articles specifically mended for urine absorption such as incontinence pads or diapers. The permeability orflow conductivity is a measure of the capability of the absorbent gelling material, following initial absorption and swelling, of effectively receiving and acquiring further amounts of liquid. This permeability or flow conductivity can be defined herein in terms of the Saline Flow Conductivity (SFC) value of the absorbent gelling material SFC in fact measures the ability of the formed hydrogel zone or layer to transport or distribute body fluids under usage pressures. For the purposes of the present invention, the Saline Flow Conductivity of the absorbent gelling material is measured according to the test described in Goldman et al., US Patent 5,599,335 issued February 4, 1997.

The SFC value of the absorbent gelling material which can be included in the odour control system of the absorbent article of the present invention is at least 35x10⁻⁷ cm³·s/g, or at least 40x10⁻⁷ cm³·s/g, or also at least 45x10⁻⁷ cm³·s/g.

According to an alternative embodiment of the present invention, the absorbent gelling material which can be comprised in the absorbent article can be selected among the polyacrylate based polymers described in the European Patent Application EP 05023061 4, filed on 21 October 2005 in the name of The Procter and Gamble Company. As explained in the referenced application, polyacrylate based materials being very slightly crosslinked, or substantially not crosslinked at all, incorporated in absorbent articles for the absorption of proteinaceous or serous body fluids such as for example menses, blood, plasma, vaginal secretions, and also mucus or milk, but particularly menses, provide an improved absorption and retention capacity for such body fluids, and an improved absorption rate as well, compared to traditional crosslinked superabsorbents.

According to the above referenced application, a measure of the degree of crosslinking of a polyacrylate based polymer can be expressed in terms of the soluble or extractable fraction of the polymer. As it is known in the art, lower molecular weight polymer chains can be solubilized, or extracted, from the polymer in certain conditions, and represent said soluble or extractable fraction of the polymer itself. Generally, the extractable fraction can be considered to be inversely proportional to the degree of crosslinking, that is, the higher the degree of crosslinking, the lower the fraction, since a greater proportion of the polymer mass is actually incorporated into the polymer network. Such polyacrylate based polymer which can be incorporated in an absorbent article for absorption of proteinaceous or serous body fluids, particularly menses, has an extractable fraction of more than 15%, or of more than 20%, or also of more than 30% by weight, wherein said extractable fraction is evaluated with the test method described herein. Desirably, said extractable fraction should be not more than 60% by weight of the polyacrylate based material, or not more than 50% by weight. The extractable fraction is evaluated according to the Extractables Test described in the above referenced European Application EP 05023061 4.

Absorbent gelling materials, for example those with the selected high permeability, as described above, or alternatively polyacrylate based absorbent gelling materials with the selected extractable fraction, provide a synergic effect in combination with the odour control system in the absorbent article of the present invention, in that they add to the activity of the odour control materials towards malodours and malodorous substances, and at the same time provide an improved capability of fluid acquisition and handling during use, also after first absorption and swelling, in particular for articles intended for absorption of urine, respectively, and for articles intended for absorption of proteinaceous or serous body fluids, typically catamenial articles for the absorption of menses.

Typically, the absorbent gelling material can be comprised in the absorbent articles of the present invention such that it is in a weight ratio with the odour control materials of the odour control system of from 10:1 to 100:1, or from 20:1 to 80:1, or also from 50.1 to 70.1.

According to an embodiment of the present invention, the absorbent gelling material can be provided in the absorbent article in a basis weight of 1 g/m² to 200 g/m², or also of 10 g/m² to 150 g/m², typically depending on the intended use of the absorbent article, as it is known in the art. The absorbent gelling material can be incorporated in the absorbent article as it is known in the art, for example typically comprised in the absorbent core.

### a) High vapour pressure materials

The action of the odour control system of the present invention can be further improved by providing the absorbent article with a highly volatile component. According to the law of Dalton, due to their high partial pressure in the headspace of the absorbent article such highly volatile components are believed to reduce the molar fraction of the malodorous compounds having a lower partial pressure.

Suitable highly volatile components include materials that have a KI (Kovat Index) below 1500 and pleasant odour. The Kovat Index is defined by the selective retention of perfume raw materials (PRMs) onto chromatographic columns. The values of the Kovat Index are obtained with a chromatographic column DB-5, 30 m, 0 25 mm, 100 µm, available from Agilent Technologies Inc (formerly J&W Scientific), operating under the following conditions 50-300°C, 4°C/min, 12.0 psi, constant flow; or equivalent equipment working under same or equivalent conditions, as can be readily determined by the man skilled in the art. A PRM's polarity, molecular weight, vapor pressure, boiling point, and the stationary phase property determine the extent of retention.

Suitable highly volatile components that act according to this mechanism can include e.g. limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and /or triethyl acetate, para-cresol and para-cymene.

### b) Solvents

Further additional ingredients include solvents as carriers for incorporating the odour control materials into the absorbent article. Suitable solvents are e.g benzyl-benzoate, isopropyl myristate, methyl abietate, ethanol, isopropanol, diethylene glycol monoethyl ether, glycerol, propylene glycol, 1,2-butylene glycol, dipropylene glycol, 2-methyl-2,4-pentanediol, diethyl phthalate, trietyl citrate, diethyl sebacate.

### The odour control system

It has been found by the present inventors that the combination of certain, separately known odour control materials results in a synergistic effect in terms of odour reduction.

Specifically, a synergistic effect in terms of malodour reduction is observed when combining odour control materials acting on the malodours or malodorous substance itself and odour control materials acting on nose receptors, as described above. In fact, an odour control system combining actives of the aforementioned two classes may reduce the malodours more efficiently than the mathematical combination of each material acting individually.

Without wishing to be bound by theory this is believed to be a result of the neutralization of the malodorous molecules in the absorbent article by the odour control materials acting internally, which reduces the concentration of these molecules in the head space and thus, in the air. Due to this, the odour control materials acting externally onto the nose receptors can exploit their maximum activity.

When the odour control system of the present invention is used in absorbent articles the individual odour control materials can be employed at variable amounts. Starting with the odour control material acting internally, for alpha-amylcinnamic aldehyde, an amount of from 0.05 g/m² to 20 g/m², preferably from 0.5 g/m² to 5 g/m² has proven useful. An example of an odour control material acting on nose receptors includes menthyl acetate, in the range from 0.05 g/m² to 20 g/m², preferably from 0.5 g/m² to 5 g/m².

The odour control system can comprise the first class of odour control material, acting on the malodours or malodorous substance, in relation to the second class of odour control material, acting on the nose receptors, at a ratio of from 50:1 to 1:50 by weight, or from 30: 1 to 1:30 by weight or also from 1.15 to 15.1 by weight. In one embodiment, the odour control system can comprise alpha-amylcinnamaldehyde and menthyl acetate at a ratio of 1:1 by weight.

### Absorbent article

The absorbent article being provided with the odour control system herein is an absorbent article of personal hygiene, specifically a disposable absorbent article selected from diapers, surgical dressings, wound dressings, perspiration pads, incontinence pads, sanitary napkins, panty liners, tampons for feminine hygiene and interlabial devices. The odour control system of the present invention can be present in any part of the absorbent article. According to the present invention the odour control system can either be present in the absorbent article as an intimate mixture of the at least two classes of odour control materials or with both classes of odour control materials being separate from each other.

In one embodiment of the present invention the odour control system can be present in the absorbent core. In another embodiment of the present invention the odour control system can be present in or on the secondary topsheet, which might be present between the topsheet, which is the uppermost layer of the absorbent article, and the absorbent core. A further embodiment of the present invention can have the first class of odour control materials (acting on the malodours or malodorous substance) placed on the wearer-facing surface of the absorbent core and the second class of odour control materials (acting on nose receptors) placed on the garment-facing surface of the absorbent core. However, other arrangements of the first and second odour control materials are contemplated including placing them in other elements of the article and/or providing one or more of them separate from the article.

### Test methods and data

For proving the synergistic effect on odour reduction several test samples have been prepared, which are all being exposed to a 0.1% aqueous solution of ammonia, which serves as a test malodorous substance. The first data point in table 2 serves as a benchmark as it represents the degree of unpleasantness of the ammonia solution without added odour control material. Data points 2-3 in table 2 are illustrating the malodour-reducing activity of two odour control materials alone, specifically alpha-amylcinnamaldehyde as aldehyde and menthyl acetate as odour control material acting on the nose receptors.

The last data point 4 in table 2 is illustrating the activity of the odour control system of the present invention. For comparability two individual odour control materials were mixed at half their amount compared to data points 2-3. Data point 4 has been obtained by testing a mixture of alpha-amylcinnamaldehyde and menthyl acetate. It is clearly obtainable from data point 4 that the odour reduction performance of the odour control system is significantly better than the one of the individual odour control materials. Thus, synergistic odour control activity has been proven.

**Table 1**

| Data point # | Sample composition | Odor unpleasantness (%) |
|---|---|---|
| 1 | Malodorant solution (10ml) | **100** |
| 2 | Alpha-Amylcinnamaldehyde (16 mg) + Malodorant solution (10ml) | **64** |
| 3 | Menthyl acetate (16 mg) +Malodorant solution (10 ml) | **46** |
| 4 | Alpha-Amylcinnamaldehyde (8mg) + Menthyl acetate (8 mg) + Malodorant solution (10 ml) | **30** |

The odour unpleasantness and pleasantness of these samples have been evaluated by a panel of expert graders. In particular five different expert graders have evaluated 4 replicates for each sample. Odour unpleasantness have been evaluated by using a scale from -10 to + 0, where -10 indicates the max odour unpleasantness, 0 indicates no odour. Data have been then reported as % relative unpleasantness vs. Reference (malodourant solution). The odour evaluation has been performed in adequate room, at controlled T (25°C). The room is equipped with appropriate conditioning system allowing continuous exchange of air. The samples were held in numbered metal trays, which were covered with aluminium foil between the actual gradings.

Each dimension for which a value is defined herein is a technical dimension, which, in the context of the present invention is not to be understood literal. Hence, all embodiments having dimensions functionally equivalent to the dimensions stated herein are intended to be covered by the scope of the invention, e.g. a dimension of "40 mm" has to be understood as meaning "about 40 mm".

## Claims

1. A disposable absorbent article selected from diapers, surgical dressings, wound dressings, perspiration pads, incontinence pads, sanitary napkins, panty liners, tampons for feminine hygiene and interlabial devices, said absorbent article comprising an odour control system, wherein the odour control composition comprises two classes of odour control material, wherein a first class of odour control material reduces odour by acting on malodours or a malodorous substance in the absorbent article and a second class of odour control material reduces odour by acting on the user's nose receptors, the absorbent article **characterized in that** the first class of odour control material is selected from aldehydes selected from the group consisting of alpha-amylcinnamic aldehyde in an amount of from 0.05 g/m² to 20 g/m², and said second class of odour control material is selected from the group consisting of menthyl acetate in an amount of from 0.05 g/m² to 20 g/m².

2. The absorbent article of claim 1, wherein the odour control system further comprises a highly volatile component selected from the group consisting of limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and /or triethyl acetate, para-cresol, para-cymene, methyl abietate and mixtures thereof.

3. The absorbent article of any of the previous claims, wherein the odour control system comprises alpha-amylcinnamaldehyde in an amount of from 0.5 g/m² to 5 g/m².

4. The absorbent article of any of the previous claims, wherein the odour control system comprises the first class of odour control material in relation to said second class of odour control material at a ratio of from 50:1 to 1:50 by weight.

5. The absorbent article of claim 4, wherein the odour control system comprises alpha-amylcinnamaldehyde and menthyl acetate at a ratio of 1:1 1 by weight.

6. The absorbent article of any of the previous claims, which further comprises an absorbent gelling material.

7. The absorbent article of claim 6, wherein said absorbent gelling material has a Saline Flow Conductivity (SFC) value of at least 35x10⁻⁷ cm³·s/g.

8. The absorbent article of claim 6, wherein said absorbent gelling material is selected among polyacrylate based materials having an extractable fraction of more than 15% by weight.

9. The absorbent article of any of claims 6 to 8, wherein the absorbent gelling material is in a weight ratio with the odour control materials of the odour control system of from 10:1 to 100:1.

10. The absorbent article of any of claims 6 to 9, wherein the absorbent gelling material is provided in a basis weight of 1 g/m² to 200 g/m².

## Patentansprüche

1. Einweg-Absorptionsartikel, ausgewählt aus Windeln, chirurgischen Verbänden, Wundverbänden, Schweißpolstern, Inkontinenzeinlagen, Damenbinden, Slipeinlagen, Tampons für die Damenhygiene und interlabialen Vorrichtungen, wobei der Absorptionsartikel ein Geruchsregulierungssystem umfasst, wobei die Geruchsregulierungszusammensetzung zwei Klassen von Geruchsregulierungsmaterialien umfasst, wobei eine erste Klasse von Geruchsregulierungsmaterialien Gerüche reduziert, indem sie auf schlechte Gerüche oder eine übelriechende Substanz im Absorptionsartikel einwirkt, und eine zweite Klasse von Geruchsregulierungsmaterialien Gerüche reduziert, indem sie auf die Nasenrezeptoren des Benutzers einwirkt, wobei der Absorptionsartikel **dadurch gekennzeichnet, dass** die erste Klasse von Geruchskontrollmaterial aus Aldehyden ausgewählt ist, die aus der Gruppe ausgewählt sind bestehend aus Alpha-Amylzimtaldehyd in einer Menge von 0,05 g/m² bis 20 g/m², und die zweite Klasse von Geruchsregulierungsmaterial aus der Gruppe ausgewählt ist bestehend aus Menthylacetat in einer Menge von 0,05 g/m² bis 20 g/m².

2. Absorptionsartikel nach Anspruch 1, wobei das Geruchsregulierungssystem ferner einen stark flüchtigen Bestandteil ausgewählt aus der Gruppe bestehend aus Limonen, Eucalyptol, Cresol, Linalool, Tetrahydrolinalool, Myrcenol, Tetrahydromyrcenol, Dihydromyrcenol, Myrcen, Cytronellol, Cytronellyilderivaten, Geraniol, Geranylderivaten, Linalylacetat, Mugetanol, Eugenol, Jasmal, Terpineol, Pinanol, Cedren, Damascon, Betapinen, Cineol und seinen Derivaten, Nonadienol, Ethylhexanal, Octanolacetat, Methylfurfural, Terpinen, Thujen, Amylacetat, Benzylacetat, Camphen, Citronellal, Dihydrocumarin, Dyhydromyrcenylacetat, Geraniol, Geranial, Isoamylacetat, Ethyl und/oder Triethylacetat, Paracresol, Paracymen, Methylabietat und Mischungen davon umfasst.

3. Absorptionsartikel nach einem der vorstehenden Ansprüche, wobei das Geruchsregulierungssystem alpha-Amylzimtaldehyd in einer Menge von 0,5 g/m² bis 5 g/m² umfasst.

4. Absorptionsartikel nach einem der vorstehenden Ansprüche, wobei das Geruchsregulierungssystem die erste Klasse von Geruchsregulierungsmaterial in Bezug auf die zweite Klasse von Geruchsregulierungsmaterial in einem Gewichtsverhältnis von 50:1 bis 1:50 umfasst.

5. Absorptionsartikel nach Anspruch 4, wobei das Geruchsregulierungssystem alpha-Amylzimtaldehyd und Menthylacetat in einem Gewichtsverhältnis von 1:1 1 umfasst.

6. Absorptionsartikel nach einem der vorstehenden Ansprüche, ferner umfassend ein Absorptionsgeliermaterial.

7. Absorptionsartikel nach Anspruch 6, wobei das Absorptionsgeliermaterial einen Flüssigkeitsweiterleitungs-(SFC)-Wert von mindestens 35x10⁻⁷ cm³·s/g umfasst.

8. Absorptionsartikel nach Anspruch 6, wobei das Absorptionsgeliermaterial ausgewählt ist aus polyacrylatbasierten Materialien mit einer extrahierbaren Fraktion von mehr als 15 Gew.-%.

9. Absorptionsartikel nach einem der Ansprüche 6 bis 8, wobei das Absorptionsgeliermaterial in einem Gewichtsverhältnis mit den Geruchsregulierungsmaterialien des Geruchsregulierungssystems von 10:1 bis 100:1 vorliegt.

10. Absorptionsartikel nach einem der Ansprüche 6 bis 9, wobei das Absorptionsgeliermaterial in einem Basisgewicht von 1 g/m² bis 200 g/m² bereitgestellt ist.

## Revendications

1. Article absorbant jetable choisi parmi couches, pansements chirurgicaux, pansements de plaies, tampons anti-transpiration, tampons pour incontinence, serviettes hygiéniques, protège-slips, tampons pour hygiène féminine et dispositifs interlabiaux, ledit article absorbant comprenant un système de régulation des odeurs, dans lequel la composition de régulation des odeurs comprend deux classes de matériaux de régulation des odeurs, dans lequel une première classe de matériau de régulation des odeurs réduit les odeurs en agissant sur les mauvaises odeurs ou une substance malodorante dans l'article absorbant et une seconde classe de matériau de régulation des odeurs réduit les odeurs en agissant sur les récepteurs nasaux de l'utilisateur, l'article absorbant **étant caractérisé en ce que** la première classe de matériau de régulation des odeurs est choisie parmi les aldéhydes choisis dans le groupe constitué d'aldéhyde alpha-amylcinnamique en une quantité allant de 0,05 g/m² à 20 g/m², et que ladite seconde classe de matériau de régulation des odeurs est choisie dans le groupe constitué d'acétate de menthyle en une quantité allant de 0,05 g/m² à 20 g/m².

2. Article absorbant selon la revendication 1, dans lequel le système de régulation des odeurs comprend en outre un composant hautement volatil choisi dans le groupe constitué de limonène, eucalyptol, crésol, linalol, tétra-hydrolinalol, myrcénol, tétra-hydromyrcénol, di-hydromyrcénol, myrcène, cytronellol, dérivés de cytronellyile, géraniol, dérivés de géranyle, acétate de linalyle, mugétanol, eugénol, jasmal, terpinéol, pinanol, cédrène, damascone, bêta pinène, cinéole et ses dérivés, nonadiénol, ethylhexanal, acétate d'octanol, méthyl furfural, terpinène, thujène, amylacétate, benzylacétate, camphène, citronellal, dihydrocoumarine, acétate de dyhydromyrcényle, géraniol, géranial, isoamylacétate, acétate d'éthyle et/ou de triéthyle, para-crésol, para-cymène, abiétate de méthyle et des mélanges de ceux-ci.

3. Article absorbant selon l'une quelconque des revendications précédentes, dans lequel le système de régulation des odeurs comprend de l'alpha-amylcinnamaldéhyde en une quantité allant de 0,5 g/m² à 5 g/m².

4. Article absorbant selon l'une quelconque des revendications précédentes, dans lequel le système de régulation des odeurs comprend la première classe de matériau de régulation des odeurs par rapport à ladite seconde classe de matériau de régulation des odeurs à un rapport allant de 50:1 à 1:50 en poids.

5. Article absorbant selon la revendication 4, dans lequel le système de régulation des odeurs comprend de l'alpha-amylcinnamaldéhyde et de l'acétate de menthyle à un rapport de 1:1 1 en poids.

6. Article absorbant selon l'une quelconque des revendications précédentes, qui comprend en outre un matériau absorbant gélifiant.

7. Article absorbant selon la revendication 6, dans lequel ledit matériau absorbant gélifiant a une valeur de conductivité de flux salin (CFS) d'au moins 35 x 10⁻⁷ cm³ s/g.

8. Article absorbant selon la revendication 6, dans lequel ledit matériau absorbant gélifiant est choisi parmi des matériaux à base de polyacrylate ayant une fraction extractible de plus de 15 % en poids.

9. Article absorbant selon l'une quelconque des revendications 6 à 8, dans lequel le matériau absorbant gélifiant est dans un rapport pondéral avec les matériaux de régulation des odeurs du système de régulation des odeurs allant de 10:1 à 100:1.

10. Article absorbant selon l'une quelconque des revendications 6 à 9, dans lequel le matériau absorbant gélifiant est fourni dans une masse surfacique allant de 1 g/m² à 200 g/m².
